# EUROPEAN PATENT APPLICATION

(11) **EP 1 361 228 A1**
(43) Date of publication of application: **12.11.2003**
(21) Application number: 02010593.8
(22) Date of filing: 10.05.2002
(51) Int. Cl.: C07K 5/083, C12Q 1/68, G01N 33/58

(54) **Thermostable and monoconjugatable gold cluster complexes**

(71) Applicant: Von Kiedrowski, Günter, 44797 Bochum-Stiepel (DE); Ruhr-Universität Bochum, 44780 Bochum (DE)
(72) Inventor: von Kiedrowski, Günter, 44797 Bochum (DE); Pankau, Wolf Matthias, 44801 Bochum (DE); Mönninghoff, Sven, 58285 Gevelsberg (DE)
(74) Representative: Helbing, Jörg, Dr. Dipl.-Chem.

(57) **Abstract**

The present invention provides a conjugatable metal cluster complex comprising a metal cluster of type Mₖ and a multivalent thioether ligand comprising at least two ligand subunits and having one reactive site or one protected reactive site which can be rendered reactive for conjugation, and each of said subunits having at least three thioether moieties, the thioether ligand, its production, and the use of the complex for PCR, labelling, fluorescence quenching and identification.

## Description

The present invention provides a conjugatable metal cluster complex comprising a metal cluster of type Mₖ and a multivalent thioether ligand comprising at least two ligand subunits and having one reactive site or one protected reactive site which can be rendered reactive for conjugation, and each of said subunits having at least three thioether moieties, the thioether ligand, its production, and the use of the complex for PCR, labelling, fluorescence quenching and identification. Moreover the the complex can be used for the labelling of molecules, especially biomolecules such as peptides, proteins, oligonucleotides and nucleic acids, lipids, sacharides and oligosacharides as well as mixed forms of the latter such as glycopeptides, glycoproteins, lipopeptides, lipoproteins, as well as functional mimics of the latter such as PNA, pRNA, threose nucleic acids, stereoisomeric peptides and nucleic acids including spiegelmers (enantiomeric forms of RNA and DNA), but also synthetic polymers, block-copolymers and dendrimers, natural products not listed above such as steroids, terpenes, alkaloids, antibiotics, vitamins, and others as well as non-natural chemicals and applications of the conjugates for imaging (TEM, STM, AFM), cytostaining, histology, immunostaining, silver staining of gels and other materials, electrochemical detection of binding events on electronic chips, fluorescence quenching in molecular beacons for quantitative PCR, nanoscale antenna for the selective inductive heating of labelled molecules by electromagnetic radiation in the MHz to THz range, most preferable around 1 GHz, allowing to control the behaviour of labelled species in the sense of a switching process e.g. based on the melting of supramolecular or non-covalent aggregates including intramolecular switching processes, as well as applications as building blocks in nanotechnology such as the production of nanowires, nanocircles and nanocoils, including novel approaches in nanorobotics based on the noncovalent nanoscaffolding of functional modules.

### Background of the Invention

The Schmid cluster complex (Schmid, G. et al., Chem. Ber., 114:3634 (1981)) Au₅₅(PPh₃)₁₂Cl₆ has stimulated many different areas of technology ranging from catalysis research (Haruta, M. et al., J. Catal., 144:175 (1993)) to the concept of quantum electronics (Volokitin, Y. et al., Nature 384:621-623 (1996)). Monofunctionalized, water soluble derivatives of the cluster complex (Hainfeld, J. F. and Furuya, F. R., J. Histochem. Cytochem., 40:177-184 (1992)) became commercially available and found numerous applications (Bendayan, M., Science, 291:1363-5 (2001)) e.g. as antibody- and peptide conjugates for TEM imaging that enabled recent discoveries in neurobiology (Bergles, D. E. et al., Nature, 405:187-190 (2000); Nusser, Z., Nature, 395:172-177 (1998); Segond von Banchet, G. and

Heppelman, B., J. Histochem. Cytochem., 43, 821 (1995); Malecki, M. et al., Proc. Natl. Acad. Sci. USA, 99:213-218 (2002); Shigemoto, R. et al., Nature, 381:523-525 (1996)). Oligonucleotide conjugates of these cluster complexes contributed to the design of nanocrystal arrays (Mirkin, C. A. et al., Nature, 382: 607-609 (1996); Alivisatos, A.P. et al., Nature 382:609-611 (1996); Niemeyer, C. M., Angew. Chem. Int. Ed. Engl., 40:4128-4158 (2001)). Recently, gold clusters were introduced as universal fluorescence quenchers in molecular beacons (Dubertret, B. et al., Nature Biotech., 19:365-370 (2001)) and as nanoscale antenna for GHz radio frequency radiation whose receival causes local and selective inductive heating of cluster-labelled biomolecules (Hamad-Schifferli, K., Nature, 415:152-155 (2002).

These articles as well as current experiments in DNA nanoscale robotics (Dorenbeck, A. et al., submitted; Eckard, L. et al., submitted) point to the need for an increased thermostability of gold cluster labels, in particular of water-soluble golg cluster complexes, which can be used as monolabels by carrying a single functional group that allows monoconjugation, which are thermostable as well as chemically stable because of a suitable ligand shell, and which are uniform in particle size due to the fact that the size of the ligand shell matches the geometric and electronic requirements of the cluster core. Possible future applications of such clusters in nanotechnology and medicine also call for a rational and tailored synthesis of ligands that allow to shield the cluster core in a defined, viz non-statistical mode of complexing thereby allowing to prepare larger quantities of the materials at an increased level of cost-efficiency.

It is known that the replacement of the monopodant triphenylphosphane ligands in such clusters against suitable functionalised tripodal thioethers based on 1,3,5-trismercaptomethylbenzene scaffolds results in gold clusters with improved stability and water solubility (Pankau, W. M. et al., Chem. Commun., 519-520 (2001)).

The problem posed in view of the above state of the art therefore is to find a ligand for metal clusters, in particular gold clusters, which form complexes with said metal clusters and which render said complexes thermally stable up to 100 °C, in particular stable under the temperature changes of the polymerase chain reaction. Further, said cluster complexes should be water soluble and have a site reactive for coupling to form conjugates with a biomolecule or any other molecule.

### Summary of the Invention

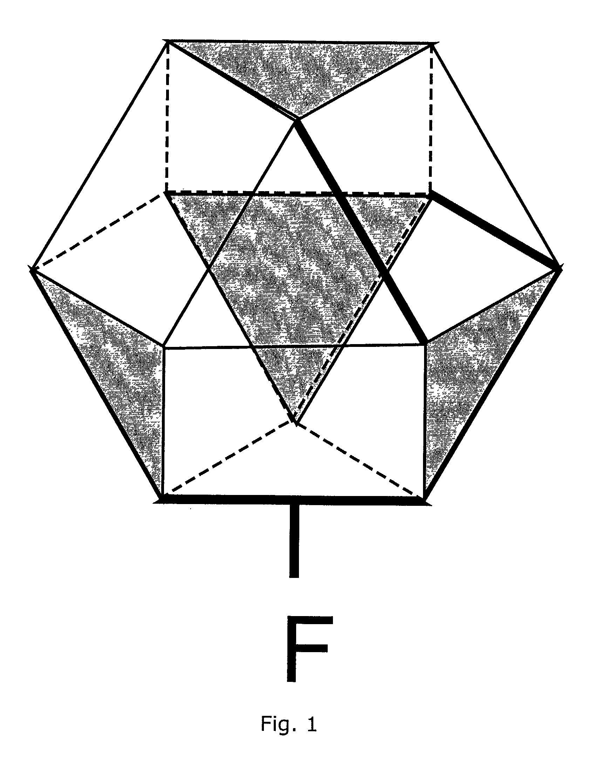

The concept behind the ligand of the present invention is sketched in Figure 1. The metal cluster core, here an Au₅₅ cluster core may be described as a cuboctahedron whose surface is composed of eight edge connected triangles (111) and six squares (110). As such, Au₅₅ is expected to bind four tripodal ligands if the binding mode of the ligands is comparable to the Schmid cluster where the 12 triphenylphosphanes most likely occupy the edges of the cuboctahedron. It was now found that if the four triangles are connected by suitable linkers, a dodekadentate monoligand results which provides for an increased stability of the complex compared to the four single tripodal ligands. Moreover it was found that by a specific synthesis it is possible to establish a the final material that carries a single functional moiety ready for bioconjugation. Moreover, the functional groups of the ligand are selected so as to render the complex sufficiently water soluble.

Therefore, the present invention provides a new generation of biocompatible gold clusters that are captured by a single, dodekadentate, thioether-based, nanoscale "grip". Moreover, the present invention demonstrates that "gripped" clusters in oligonucleotide conjugates survive the temperature conditions of PCR and hybridisation experiments.In particular, the present invention relates to
(1) a conjugatable metal cluster complex comprising
   (a) a metal cluster of type Mₖ, and
   (b) (b) multivalent thioether ligand comprising at least two ligand subunits and having one reactive site or one protected reactive site which can be rendered reactive for conjugation, and each of said subunits having at least three thioether moieties;
(2) a preferred embodiment of the cluster as defined in (1) aboveis that the ligand has the linear structure (I)

   (BₘAB'ₙ)ₚ(BₘAB'ₙ)(BₘAB'ₙ)_{q} (I)

   or the cyclic structure (II) 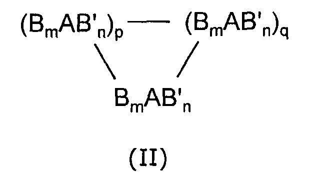 or the dendrimeric structure (III) 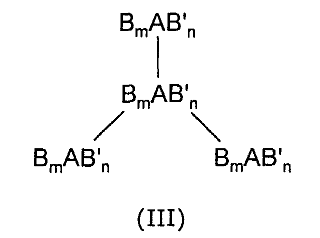 wherein (BₘAB'ₙ) corresponds to one subunit, wherein
   (i) A is a core structure selected from substituted or unsubstituted aryl, heteroaryl, cycloalkane or heterocycloalkane residue, or a carbon atom, nitrogen atom, preferably said core structure has C₃ symmetry and more preferable is a substituted or unsubstituted benzene residue, even more preferable the core structure has attached thereto the at least three thioether moieties and/or linear or branched alkyl moieties being directly or through the thioether attached to the core structure; and
   (ii) B and B' are substituted by one or more first functional groups selected from -COOH, -COOR', -OP(O)(OH)₂, -OP(O)(OH)OR', -OP(O)(OR')₂, -SH, -SO₂R', -SO₃H, and -SO₃R' (where R' is a protecting or leaving group); and
   (iii) B' is further substituted by at least one second functional group which is selected from -OH, -SH, -NH₂ and a protected form thereof, and preferably is -NH₂ or a protected -NH₂ group, whereby said second functional group of one B' of the ligand forms the reactive site or protected reactive site which can be rendered reactive for conjugation; and
   (iv) m, n, p, q are integers each ranging independently from 0 to 25, preferably from 0 to 10, whereby in case of the linear structure (I) m + n ≥ 3 and p + q ≥ 1 and in case of the cyclic structure (II) m + n ≥ 3 and p + q ≥ 2, preferably in any case m + n = 3 and p + q = 3; and/or
   (v) at least two subunits are bonded to each other through a covalent B-B' linkage which is achieved by reaction of the first functional group of B with the second functional group of B';
(3) in an even more preferred embodiment of the cluster as defined in (1) and (2) above is that the subunit corresponds to the formula (IV) 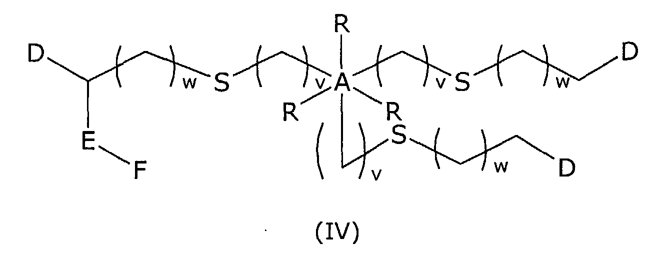 wherein,
   (i) A is the core structure as defined in claim 3, preferably is a 1,3,5 trisubstituted benzene optionally having 1 to 3 additional substituents R; and
   (ii) R is independently selected from H or lower alkyl, preferably any of H, -CH₃, -C₂H₅, halogen; and
   (iii) D is the first functional group as defined in claim 3; and
   (iv) E is the second functional group as defined in claim 3; and
   (v) F is H or a protecting group and for one subunit of the ligand F is H or a protecting group or a functional group or a moiety which can be rendered functional selected from from -NH₂, -CHO, -OH, -Halogen, -SH, and -SS so that the E-F moiety forms the reactive site or protected reactive site which can be rendered reactive for conjugation; and
   (vi) v and w are integers each ranging independently from 0 to 10, preferably is 1;
(4) a most preferred embodiment of the cluster as defined in (1) to (3) above is that the ligand has the structure of formula (VI): 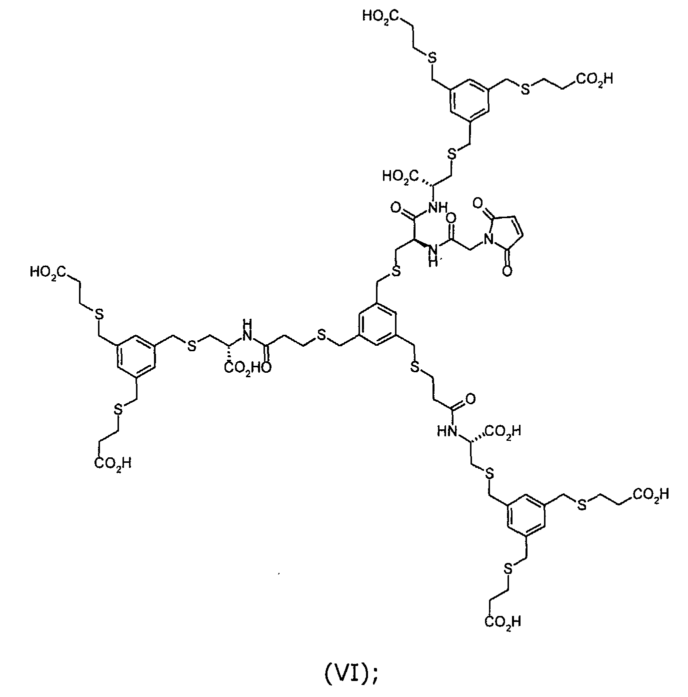
(5) the ligand as defined in (1) to (4) above or the subunit as defined in (3) above;
(6) a method for preparing the ligand as defined in (3) or (4) above, which method comprises the following steps of
   (i) preparing the subunits as defined in claims 4 or 5; and
   (ii) coupling together at least two subunits obtained in step (i), whereby one of the subunits coupled together has a reactive site or protected reactive site which can be rendered reactive for conjugation; and
   (iii) optionally deprotection;
(7) the use of the complex as defined in (1) to (4) above in a polymerase chain reaction (PCR), for labelling of a carbohydrate, peptide, nucleotide, peptide-nucleotide-adduct (PNA), polyether, for quenching the fluorescence of one or more fluorescence labels, for the identification of a nucleotide, peptide-nucleotide-adduct (PNA) or oligonucleotide, as TEM-Labels, for immunostaining, etc.

### Description of the Figures

- Fig. 1: General concept of a gold cluster grip. Four tripodal binders employing thioethers of 1,3,5-trismercaptomethylbenzene scaffolds occupy four (111) faces of the cuboctahedral cluster. The triangles are linked (bold lines) to result in a dodekadentate monoligand carrying a functional group F that allows biomolecule monoconjugation.

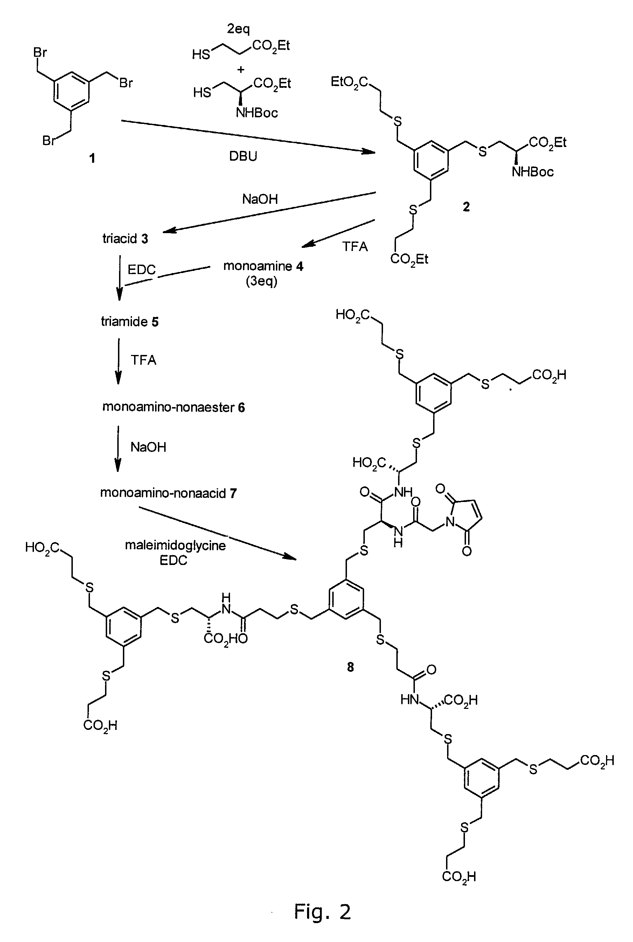

- Fig. 2: Structure and synthesis of the dodekadentate grip 8 carrying a monomaleimido moiety.

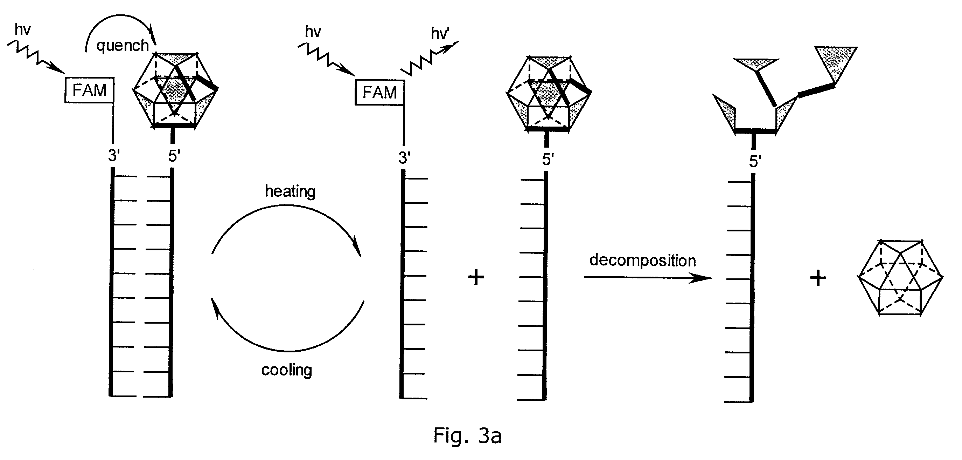

- Fig. 3a: Chemical and physical processes underlying the determination of cluster stability in a cluster torturing experiment. Cluster torturing involves cycles of heating and cooling while measuring the fluorescence of a 3'-fluoresceine labelled oligonucleotide in the presence of the 5'-gold labelled complement.

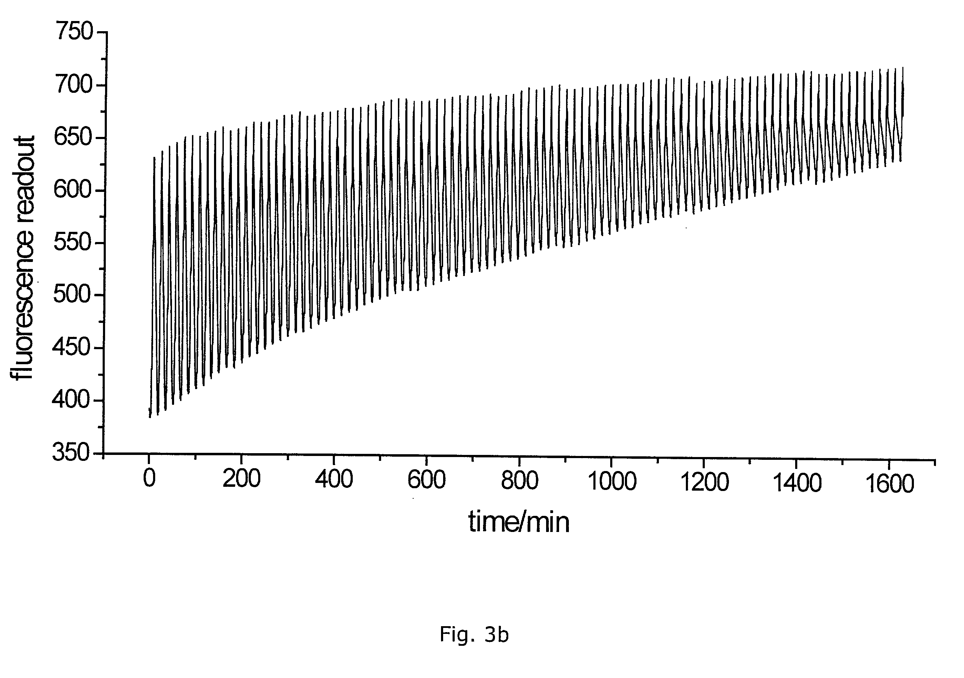

- Fig. 3b: Fluorescence readout during 100 cycles of torturing a solution of 1 µM 5'-ATTCCCGGTT ATGTCCAATG GGTGCAT-3'-FAM, 3 µM 5'-(6-mercaptohexylphosphate)-modified 5'-ATGCACCCAT TGGACATAAC CGGGAAT conjugated with a mixture of gold-filled and unfilled grips 8, 300 mM NaCl, 100 mM MOPS, pH 7.5.

### Detailed Description of the Invention

### In a particular embodiment of the present invention

the target ligand 8 together with a sketch of its synthesis is shown in Figure 2. The inventors of the present invention selected a monomaleimido moiety as the monoconjugable unit in order to gain functional comparability with the commercially available variant of the Schmid-cluster. Starting from trisbromide 1, trithioether 2 was generated as a key percursor that allowed to synthesize 8 on a divergent-convergent route. Ligand 8 was converted into its gold cluster by phase transfer synthesis as previously described for tris-thioethers having a structural skeleton similar to that of 2. The cluster revealed a uniform size distribution with an average diameter of 1.4 nm in high resolution TEM. The cluster was conjugated with a 5'-thiol modified 27-mer oligonucleotide, whose complement was synthesized in its 3'-fluoresceine labelled form.
For the study of thermostability of the label a "cluster torturing" experiment was performed, in which the fluorescence signal from a mixture of both oligonucleotides was monitored during 100 cycles of temperature variation. Each cycle consisted of a heating period from 20 °C to 95 °C with 10 K/min, a "torture" period for 6 min at 95 °C and a recooling period with 20 K/min giving an average temperature of 70.5 °C. The rationale of temperature cycling is depicted in Figure 3a. At low temperatures the duplex holds the gold cluster and the fluorescent dye within spatial proximity. Quenching of fluorescence takes place resulting in a low fluorescence readout. At high temperatures the fluorescent oligonucleotide exists in its single-stranded form giving an increase of the fluorescence signal due to the loss of its neighbouring quench. If the gold cluster escapes its grip during a cycle, or, if the latter's conjugation with the complement strand breaks, there will be no quench in the next cycle. Thus, kinetic information on the stability of clusters and/or their grip conjugation can be derived from the low-temperature fluorescence increase as a function of the cycle number relating to the torturing time. Figure 3b depicts how the temperature-driven fluorescence oscillations develop as a function of the time. From a comparison of experiments involving the empty and the filled grip we conclude that not the grip itself but the gold cluster inside is the reason for the quench, as to be expected for the UV absorption properties of the grip. Moreover, the "baseline" fluorescence of both, the duplex and the single strand, decrease with the temperature in each cycle, the latter however at a higher level of fluorescence intensity due to a poorer stacking of the 3'-fluoresceine to the single strand. Different baseline courses for single and double-stranded species also explain why a slight increase of fluorescence is observed for the duplex with empty grip at the melting transition.

Any conceivable approach in DNA bio- and nanotechnology, where the remarkable properties of gold clusters are to be employed will call for label compatibility with at least the basic procedures of molecular biology. To the best of the inventors' knowledge the gripped cluster presented here is the first example that an Au₅₅ monolabel survives the temperature conditions of PCR and hybridisation protocols. A typical PCR-experiment may be equivalent to perhaps 100 min of the "torturing" described in the present invention, if one recalls that the exposure to a temperature of 95 °C needs 1 min or less in PCR while each of the "torturing" cycles employed 6 min at 95 °C. The average temperature in the "torturing" experiment (see example 10) is close to PCR, so that one cycle in said experiment may be compared to 4-6 typical PCR-cycles. From Figure 3b it is evident, that only a small fraction (less than 10%) of gold nanocrystals did not survive during the first 100 min of treatment. Needless to say that the full potential of universal quenching (e.g. quantitative PCR of gene sets employing beacon sets with different dyes in the same tube) and radio frequency induced single-molecule heating (e.g. for nanoscale robotics) can only launch from a thermostable ground.

The invention is further explained by the following examples which are, however not to be construed to limit the invention.

### Examples

### Acronyms:

- δ: chemical shift (in ppm)
- DBU: 1,8-diazabicyclo[5.4.0]undec-7-ene
- EDC: 1-(3-dimethylaminopropyl)-3-ethylcarbodiimide hydrochloride
- TFA: trifluoroacetic acid
- pRNA: Pyranolsyl RNA
- AFM: Atomic Force Microscopy
- STM: Scanning Tunnel Microscopy
- PCR: Polymerase Chain Reaction
- DBU: 1,8-Diazabicyclo[5.4.0]-7-undecen
- TEA: Triethylamine
- HOBT: Hydroxybenzotriazole
- MOPS: Morpholinopropiosulfonic acid

### Example 1: Synthesis of N-Butyloxycarbonyl-S-(3,5-bisethoxycarbonylethylthiomethylbenzyl)-cysteine 2

3.83 g (28.53 mmol) 3-mercaptopropionic acid ethyl ester, 3.57 g (14.27 mmol) *N-tert*-butyloxycarbonyl-L-cysteine ethyl ester and 6.51 g (42.80 mmol) DBU were dissolved in 100 ml toluene and the solution was added dropwise over a period of 2 h to a solution of 4.63 g (12.97 mmol) tris-bromomethyl-benzol 1 in 50 ml toluene. The mixture was stirred for 2 h at ambient temperature. The precipitate was separated by filtration and washed with toluene. Excess DBU was removed by extraction with 50 ml of hydrochloric acid (5%). The toluene layer was successively washed with 50 ml of sat. NaHCO₃ and NaCl, dried over MgSO₄ and evaporated to give 9.58 g of crude material. Product 2 was isolated in 48% yield by chromatography on 750 g silica using cyclohexane/ethyl acetate = 6:1 (v/v) as eluent. ¹H-NMR (200 MHz,CDCl₃): δ = 1.26 (m, 9H), 1.46 (s, 9H), 2.61 (m, 8H), 2.68 (m, 2H), 3.70 (s, 6H), 4.16 (m, 6H), 4.48 (br. m, 1H), 5.33 (br. d, 1H), 7.15 ppm (s 3H). ¹³C-NMR ( 50 MHz, CDCl₃): δ = 14.31, 26.52, 28.45, 33.98, 34.61, 36.18, 36.58, 53.35, 60.67, 61.81, 80.19, 128.34, 138.56, 138.98, 155.28, 171.18, 171.93 ppm. MS (MALDI-TOF) m/e = 656.32 (M⁺+Na).

### Example 2: Synthesis of N-Butyloxycarbonyl-S-(3,5-biscarboxyethyl-thiomethyl-benzyl)-cysteine 3

566 mg (0.90 mmol) 2 were dissolved in 30 ml warm ethanol combined with 20 ml 1N NaOH and stirred for 2 h at ambient temperature. The pH was adjusted to 4-5 with approx. 20 ml 1N HCI and a little amount of aq. sodium bicarbonate. The organic solvent was removed by evaporation at reduced pressure and the resulting mixture was cooled to 0 °C, acidified (to pH 1) with dilute HCI until no more precipitate was formed, and extracted three times with 25 ml dichloromethane each. The combined extracts were washed with NaCl and dried over MgSO₄. Evaporation of the solvent and drying in vacuo yielded 400mg (81 %) of a colourless, hygroscopic foam. ¹H-NMR (200MHz, CDCl₃): δ = 1.43 (s, 9H), 2.55 (d, 4H), 2.62 (d, 4H), 2.58 (dd, 2H), 3.70 (s, 6H), 4.50 (br, 1H), 5.44 (m, 1H), 7.16 (s, 3H), 9.94 ppm (br, 3H). ¹³C-NMR (50 MHz, CDCl₃) δ 26.01, 28.46, 33.31, 34.55, 36.25, 53.00, 80.89, 128.51, 138.52, 139.09, 155.81, 175.76, 177.42 ppm. MS (FAB): m/e 548 (M⁺), 570 (M⁺+Na).

### Example 3: Synthesis of S-(3,5-Bisethoxycarbonylethylthiomethyl-benzyl)-cysteine 4

A stirred solution of 1.80 g (2.85 mmol) 2 in 20 ml dichloromethane to which 20 ml TFA was added dropwise, was kept for 1 h at 0 °C and 1 h at ambient temperature. Dichloromethane was evaporated in vacuo and TFA was removed by subsequent coevaporation with six 30 ml portions of chloroform. The product (1.84 g, 99%) was pure enough for further synthetic transformations. ¹H-NMR (400MHz, CDCl₃): δ = 1.25 (t, 6H), 1.29 (t, 3H), 2.54 (t, 4H), 2.66 (t, 4H), 3.05 (d, 2H), 3.69 (s, 4H), 3.74 (s, 2H), 4.10 (m, 5H), 4.15 (q, 2H), 7.15 (s, 2H), 7.20 (s, 1H), 7.67 ppm (br, 3H). ¹³C-NMR (100 MHz, CDCl₃): δ = 13.95, 14.19, 26.52, 31.62, 34.62, 36.16, 36.46, 52.69, 61.15, 63.62, 52.69, 61.15, 63.62, 128.31, 128.85, 137.77, 139.62, 167.68, 172.61 ppm. MS (FAB): m/e 532 (M⁺).

### Example 4: Synthesis of Triamide 5

A suspension of 969 mg (1.50 mmol) 4, 605 mg (6.00 mmol) TEA and 270 mg (2.00 mmol) HOBT in 10 ml absolute trichloromethane was added while stirring to a solution of 274 mg (0.50 mmol) 3 in 10 ml absolute CDCl₃. Stirring was continued until complete dissolution of the all solids. The reaction was started by the addition of 720 mg (3.75 mmol) EDC and allowed to proceed while stirring for 16 h at ambient temperature under a dry atmosphere (argon). The mixture was diluted with 100 ml trichloromethane, washed with 20 ml portions of 1 N HCI, sat. NaHCO₃ and sat. NaCl, dried over MgSO₄, and evaporated to give 1.32g of crude product, from which 5 was isolated by flash-chromatography on 100 g silica eluting with cyclohexane/ethyl acetate 1:1 (v/v). Yield: 546 mg (52%). ¹³C-NMR (50 MHz, CDCl₃): δ = 14.36, 21.15, 26.65, 27.06, 27.18, 28.53, 33.11, 34.19, 34.71, 36.27, 51.90, 60.50, 60.81, 61.99, 128.35, 138.57, 139.04, 170.96, 171.15, 171.97 ppm. MS (MALDI-TOF): m/e 2114.63 (M⁺+Na).

### Example 5: Synthesis of Monoamino nonaester 6

600 mg (0.29 mmol) of 5 were dissolved in 20 ml dichloromethane and treated with 20 ml TFA as described for 4. After work up the product (605 mg, 100%) had sufficient purity for the next synthetic step. ¹³C-NMR (50 MHz, CDCl₃): δ = 14.15, 14.27, 26.54, 33.79, 34.23, 34.65, 36.15, 52.16, 61.02, 62.35, 89.70, 128.29, 128.47, 138.24, 138.38, 139.07, 139.66, 169.93, 170.64, 172.38 ppm.

### Example 6: Synthesis of Monoamino nonaacid 7

546 mg (0.26 mmol) 6 were dissolved in 20 ml warm ethanol during sonification for 15 min. 20 ml of aq. NaOH (10%) were added and the resulting mixture was stirred for 1h at ambient temperature. The alcohol was removed by evaporation. 1N HCI was added dropwise to the remaining, vigorously stirred suspension until pH 2 was reached. The precipitate was isolated by centrifugation, washed several times with distilled water and dried in vacuo over KOH for a period of 16h to give 286 mg (63%) of the product as an colourless powder. MS (MALDI-TOF): m/e 1736.07 (M⁺).

### Example 7: Synthesis of Grip 8

17 mg (10 µmol) 7 and 25 mg (100 µmol) maleimidoglycine-NHS-ester were dissolved in 2ml DMF and stirred for 27h at 60°C. After cooling to room temperature, 20 ml of diethyl ether was added to precipitate the product, which was obtained quantitatively after centrifugation, washing with two 8 ml portions of diethyl ether and drying in a speedvac concentrator. MS (MALDI-TOF): m/e 1875.41 (M⁺), 1897.41 (M⁺+Na), 1920.16 (M⁺+2Na).

### Example 8: Phase Transfer Synthesis of Gripped Gold Clusters

A solution of 14 mg (1 µmol) of Au₅₅(PPh₃)₁₂Cl₆ in 10 ml dichloromethane was vigorously stirred for 16 h with an overlaying solution of 3.6 mg (2 µmol) of 8 in 10 ml 0.1 M potassium phosphate buffer, pH 7.0. The phases were separated by short centrifugation and the aqueous layer was aliquotated into Eppendorf tubes and evaporated using a speed vac concentrator.

### Example 9: Preparation of Oligonucleotide Conjugates

A 5'-SH modified 27mer oligonucleotide, sequence 5'-ATGCACCCAT TGGACATAAC CGGGAAT was reacted with a tenfold excess of gripped clusters in accordance to a previously established procedure developed for a commercially available monomaleimido derivative of the Schmid cluster. The reaction took place for 2 h at room temperature under an argon atmosphere.

### Example 10: Torturing experiments

The experiments were performed with the raw material from the previous protocols, meaning that a maximum of 50% of the grip conjugates can be filled with gold nanocrystals, and consequently, that the quench of fluoresceine at low temperatures can only reach a maximum of 50% of its theoretical value. The experiments were performed on a Varian Eclipse equipped with a temperature profiling control and a magnetic cuvette stirrer.

## Claims

1. A conjugatable metal cluster complex comprising
(a) a metal cluster of type Mₖ, and
(b) multivalent thioether ligand comprising at least two ligand subunits and having one reactive site or one protected reactive site which can be rendered reactive for conjugation, and each of said subunits having at least three thioether moieties.

2. The complex of claim 1 wherein
(i) the metal cluster Mₖ is a cluster wherein M is selected from one or more transition metals, heavy main group metals, in particular noble metals such as Pt, Pd, Ag, including Hg, most preferable is Au, and k is an integer ranging from 10 to 600, preferable is 55; and/or
(ii) the thioether ligand comprises at least three, preferably at least four subunits; and/or
(iii) the subunits are connected through at least one linkage selected from -C(O)O-, -C(O)S-, -C(S)O-, -C(S)S-, -C(O)NH-, -S(O)₂O-, -S(O)₂S-, -S(O)₂NH-, -O-, -S-, -S-S-, -C-C-, -C=C-, -C=N- or -C=N-NH-, preferably a -C(O)NH- moiety; and/or
(iv) the subunits are connected in a linear, cyclic or dendrimeric manner; and/or
(v) the reactive site or protected reactive site which can be rendered reactive for direct conjugation is selected from -NH₂, -OH, -SH, -Halogen, and preferable is -NH₂, or for modification and transformation into a monoconjugatable moiety such as an aldehyde, an active ester, a thioester, a hydrazide, a semicarbazide, a phosphoramidite, a vinylsulfone, a isocyanate, a isothiocyanate, a reactive disulfide, preferrable a maleimide, or for transformation into a polymerizable moiety such as an acrylamide or bis- or trisvariants of the moieties listed above; and/or
(vi) the subunit has C₃ symmetry; and/or
(vii) the ligand is in a protected form and attached as a side chain to a suitable protected amino acid, such as a N-Boc or a N-Fmoc amino acid that allow to synthesize peptide conjugates of the grip ligand or oligomers of the grip ligand or combinations of the latter units using standard peptide synthesis protocols and a postsynthetic formation of monomeric or oligomeric gold clusters after deprotection.

3. The complex of claim 1 or 2, wherein the ligand has the linear structure (I)
(BₘAB'ₙ)ₚ(BₘAB'ₙ)(BₘAB'ₙ)_{q} (I)
or the cyclic structure (II) 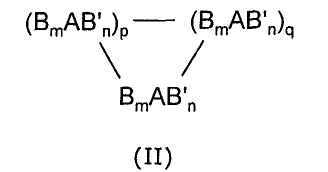 or the dendrimeric structure (III) 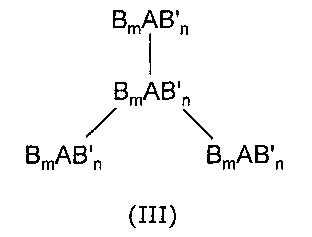 wherein (BₘAB'ₙ) corresponds to one subunit, wherein
(i) A is a core structure selected from substituted or unsubstituted aryl, heteroaryl, cycloalkane or heterocycloalkane residue, or a carbon atom, nitrogen atom, preferably said core structure has C₃ symmetry and more preferable is a substituted or unsubstituted benzene residue, even more preferable the core structure has attached thereto the at least three thioether moieties and/or linear or branched alkyl moieties being directly or through the thioether attached to the core structure; and
(ii) B and B' are lower alkyl or (lower)alkoxy(lower)alkyl moieties substituted by one or more first functional groups selected from -COOH, -COOR', -OP(O)(OH)₂, -OP(O)(OH)OR', -OP(O)(OR')₂, -SH, -SO₂R', -SO₃H, and -SO₃R' (where R' is a protecting or leaving group); and
(iii) B' is further substituted by at least one second functional group which is selected from -OH, -SH, -NH₂ and a protected form thereof, and preferably is -NH₂ or a protected -NH₂ group, whereby said second functional group of one B' of the ligand forms the reactive site or protected reactive site which can be rendered reactive for conjugation; and
(iv) m, n, p, q are integers each ranging independently from 0 to 25, preferably from 0 to 10, whereby in case of the linear structure (I) m + n ≥ 3 and p + q ≥ 1 and in case of the cyclic structure (II) m + n ≥ 3 and p + q ≥ 2, preferably in any case m + n = 3 and p + q = 3; and/or
(v) at least two subunits are bonded to each other through a covalent B-B' linkage which is achieved by reaction of the first functional group of B with the second functional group of B', or a linkage between B and B' obtained by reaction with one ore more additional bisfunctional linker molecules.

4. The complex of claim 3, wherein the subunit corresponds to the formula (IV) 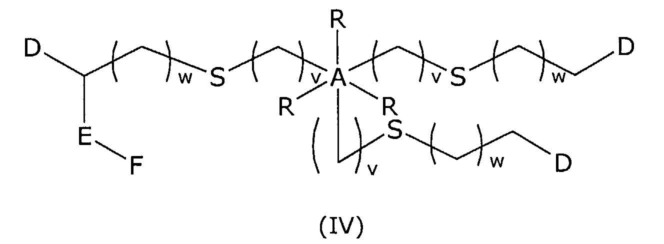 wherein,
(i) A is the core structure as defined in claim 3, preferably is a 1,3,5 trisubstituted benzene optionally having 1 to 3 additional substituents R; and
(ii) R is independently selected from H or lower alkyl, preferably any of H, -CH₃, -C₂H₅, halogen; and
(iii) D is the first functional group as defined in claim 3; and
(iv) E is the second functional group as defined in claim 3; and
(v) F is H or a protecting group and for one subunit of the ligand F is H or a protecting group or a functional group or a moiety which can be rendered functional selected from from -NH₂, -CHO, -OH, -Halogen, -SH, and -SS so that the E-F moiety forms the reactive site or protected reactive site which can be rendered reactive for conjugation; and
(vi) v and w are integers each ranging independently from 0 to 10, preferably is 1.

5. The complex of claim 4, wherein
(i) A is a benzene residue; and
(ii) R is hydrogen; and
(iii) D is COOH; and
(iv) E is -NH₂ and F is H and for one subunit of the ligand F is a moiety of formula (V) 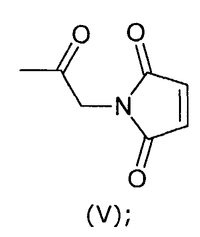 or an ω-aminocarboxylic acid or an α,ω-diaminocarboxylic analogue of the moiety of formula (V), or any other NH₂ protecting group defined in claim 2, or E-F represents any other protected functional group defined in claim 2; and
(v) v = 1 and w = 1; and
(vi) p = 1 and q = 2; and
(vii) the subunits are connected in a dendrimeric manner; and
(viii) the metal cluster preferably is Au₅₅.

6. The complex of any of claims 1 to 5 which has a ligand which has a subunit additionally comprising a first carbohydrate, peptide, nucleotide, peptide-nucleic acid (PNA), p-RNA, or polyether, preferably an oligonucleotide attached to the reactive site through a covalent bond.

7. The complex of claim 1 wherein the ligand has the structure of formula (VI): 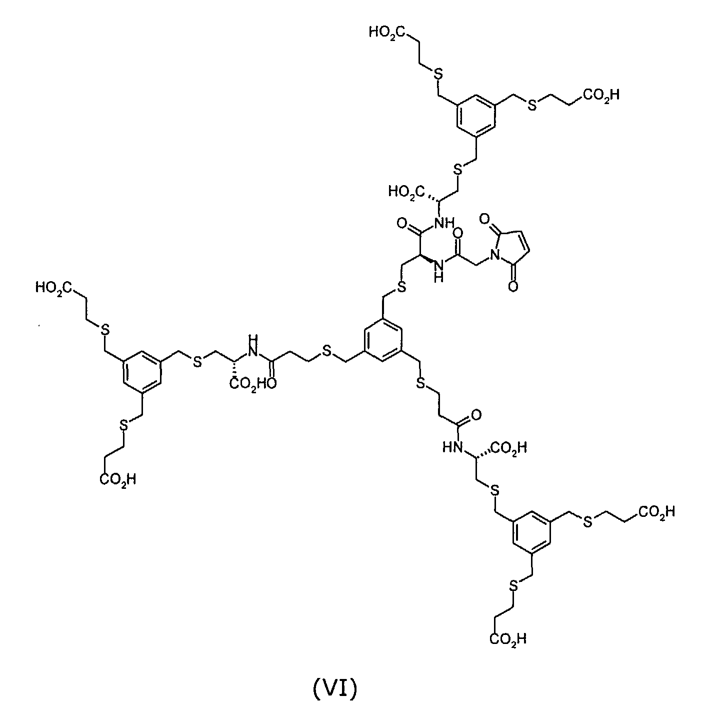 or derivatives thereof having substituents R at the benzene cores as defined in claim 4, and dia- and enantio-stereoisomeres thereof.

8. The ligand as defined in any of claims 1 to 7 or the subunit as defined in any of claims 4 or 5.

9. A method for preparing the ligand as defined in claims 4 or 5, which method comprises the following steps of
(i) preparing the subunits as defined in claims 4 or 5; and
(ii) coupling together at least two subunits obtained in step (i), whereby one of the subunits coupled together has a reactive site or protected reactive site which can be rendered reactive for conjugation; and
(iii) optionally deprotection.

10. The method of claim 9, which further comprises the steps of
(i) reacting a compound of formula (VII) 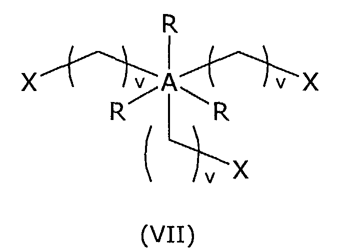 wherein
A, R, and v are as defined in any of the preceeding claims, and X is a leaving group, with the compounds of formula (VIII) and (IX) separately or with a mixture of the compounds of formula (VIII) and (IX), 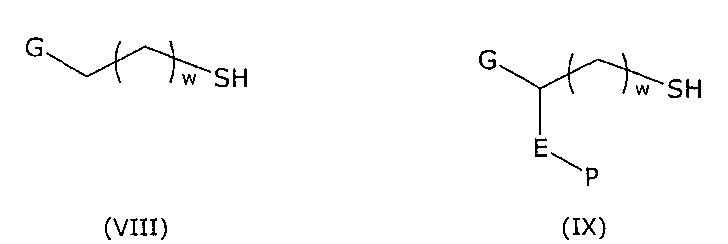 wherein w and E are as defined in any of the above claims, G is -COOH, -COOR', -OP(O)(OH)₂, -OP(O)(OH)OR', -OP(O)(OR')₂, -SH, -SO₂R', -SO₃H, -SO₃R' (where R' is a protecting or leaving group), and P is a protecting group to obtain the compound of formula (X) 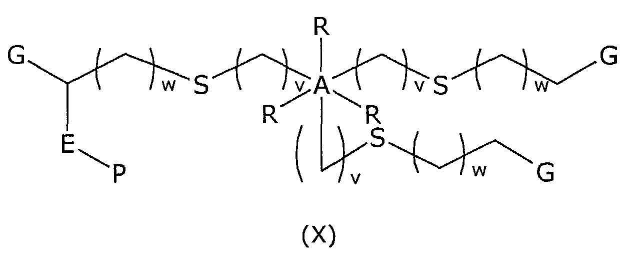 wherein A, R, E, G, P, v, and w are as defined above; and
(ii) reacting the compound of formula (X) with a suitable base or acid to obtain the compound of formula (XI) 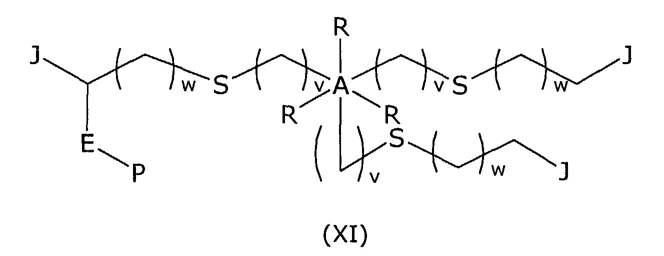 wherein A, R, E, P, v, and w are as defined above and J is selected from any of -COOH, -OP(O)(OH)OR', -SH, and-SO₃H; and
(iii) reacting the compound of formula (X) with a suitable base or acid to obtain the compound of formula (XII) 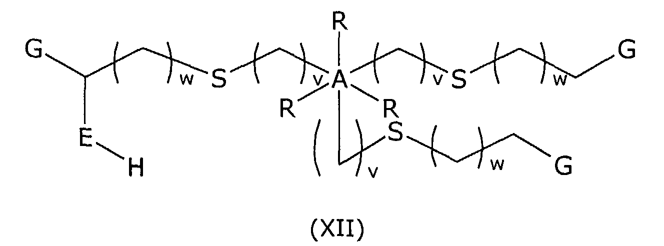 wherein A, R, E, G, v, and w are as defined above; and
(iv) reacting the compound of formula (XI) with the compound of formula (XII), preferably in a molar ratio of (XI):(XII) ≥ 1:3, and in the presence of one or more suitable activating reagents; and
(v) reacting the compound obtained in step (iv) with a reagent suitable to remove the protecting group P; and
(vi) reacting the compound obtained in step (v) with a suitable base or acid to convert all G-groups to J-groups whereby the G- and J-groups are as defined above; and
(vii) optionally reacting the free E-H group of the compound obtained in step (vi) with a reagent of formula F-Y or F-H, whereby F is as defined above but not H and Y is a suitable leaving group, and, in case F-H is employed, in the presence of a suitable water removing agent, to obtain the ligand as defined in claim 4, or a compound, wherein J is different from D as defined in claim 4; and
(viii) in case for the compound obtained in step (vii) J is different from D as defined in claim 4, all J-groups of said compound are converted to D-groups to obtain the ligand as defined in claim 4.

11. A method for preparing the subunit as defined in claim 7, which method comprises steps (i) and (ii) or steps (i) and (iii) as defined in claim 10.

12. A method for preparing the complex as defined in any of claims 1 to 7, comprising reacting the ligand with the metal cluster.

13. The method of claim 12 which further comprises reduction of a precursor for the metal cluster, preferably HAuCl₄, manipulation of conjugates by inductive heating using radio frequency in the presence of the ligand thus forming the cluster.

14. Use of the complex as defined in any of claims 1 to 7 in a polymerase chain reaction (PCR), for labelling of a carbohydrate, peptide, nucleotide, peptide nucleic acid (PNA), p-RNA, polyether, for quenching the fluorescence of one or more fluorescence labels, for the identification of a nucleotide, peptide-nucleotide-adduct (PNA) or oligonucleotide, as TEM-Labels, for immunostainingetc.


SEQUENCE LISTING

<110> von Kiedrowski
      Ruhr-Universität Bochum

<120> Thermostable and Monocojugatable Gold Cluster Complexes

<130> 021147ep/JH/HR/ml

<140>
<141>

<160> 2

<170> PatentIn Ver. 2.1

<210> 1
<211> 27
<212> DNA
<213> Artificial Sequence

<220>
<223> Description of Artificial Sequence: Primer

<400> 1
atgcacccat tggacataac cgggaat                                     27


<210> 2
<211> 27
<212> DNA
<213> Artificial Sequence

<220>
<223> Description of Artificial Sequence: Primer

<400> 2
attcccggtt atgtccaatg ggtgcat                                     27